# EUROPEAN PATENT APPLICATION

(11) **EP 4 821 945 A1**
(43) Date of publication of application: **14.10.2026**
(21) Application number: 24899514.4
(22) Date of filing: 28.10.2024
(51) Int. Cl.: C07K 16/28, C12N 15/13, A61K 39/395, A61P 31/12, A61P 37/02

(54) **ANTI-BDCA-2 ANTIBODY, PREPARATION METHOD THEREFOR AND USE THEREOF**

(30) Priority: 05.12.2023 CN 202311652149
(71) Applicant: Suzhou Pro-Heal Pharmaceuticals Technology Co., Ltd., Suzhou, Jiangsu 210000 (CN)
(72) Inventor: LIANG, Xiao, Suzhou, Jiangsu 210000 (CN); FENG, Wansheng, Suzhou, Jiangsu 210000 (CN); GUO, Shuhua, Suzhou, Jiangsu 210000 (CN); MA, Lin, Suzhou, Jiangsu 210000 (CN)
(74) Representative: Winter, Brandl - Partnerschaft mbB
(86) International application number: PCT/CN2024/127585
(87) International publication number: WO 2025/118871

(57) **Abstract**

An anti-BDCA-2 antibody, a preparation method therefor, and a use thereof, relating to the field of antibodies. The preparation method for the anti-BDCA-2 antibody comprises: carrying out ELISA detection and screening on a hybridoma cell culture supernatant and an antigen protein to obtain a positive clone, and subcloning the positive clone to obtain the anti-BDCA-2 antibody. The anti-BDCA-2 antibody comprises a heavy chain and a light chain, wherein a variable region of the heavy chain and a variable region of the light chain are selected from amino acid sequences as shown in SEQ ID NOs: 1-22. The anti-BDCA-2 antibody and related drugs can be used for inhibiting the production of IFN-α by pDCs and preventing the over-activation of the pDCs. The anti-BDCA-2 antibody can effectively inhibit the activation of pDC cells and the production of IFN-α, and has great potential in resisting virus infection and preventing certain autoimmune diseases.

## Description

The present invention claims the priority of the Chinese patent application filed with the Chinese Patent Office on December 05, 2023, with application number 2023116521493 and title "Anti-BDCA-2 antibody, preparation method therefor and use thereof", the entire contents of which are incorporated by reference herein.

### Technical field

The present invention belongs to the technical field of antibody, and specifically relating to an Anti-BDCA-2 antibody, preparation method therefor and use thereof.

### Background Art

Blood dendritic cell antigen 2 (BDCA-2) is a type II C lectin that plays an important role in the human immune system. BDCA-2 is expressed with high specificity in plasmacytoid dendritic cells (pDCs). pDCs are a special type of cell in the immune system. They are widely distributed in lymphatic and non-lymphatic tissues and are powerful interferon-producing cells. They make important contributions to innate and adaptive immunity, and play a key role especially in the early stages of viral infections and autoimmune diseases.

BDCA-2 expression on the surface of pDC can regulate the function of pDC. As an endocytic receptor, one of the main functions of BDCA-2 is endocytosis. Through endocytosis, BDCA-2 can capture and internalize antigens, further promoting antigen presentation, which is crucial for immune responses. Therefore, BDCA-2 helps the human immune system fight pathogens by participating in the antigen extraction and processing. However, unlike other endocytic receptors, the signal of BDCA-2 may also inhibit pDC from producting interferon. Once BDCA-2 is activated, it blocks interferon production by inhibiting the signaling of Toll-like receptors 7 and 9 (TLR7 and TLR9), which are key receptors that drive interferon production during viral infection. In summary, the main functions of BDCA-2 include: participating in the internalization and processing of antigens, and making fine adjustments to the disease response by inhibiting the response of pDC to interferon production.

During viral infection, BDCA-2 inhibits the generation of IFN-α by pDC by inhibiting the signal pathway of TLR9, thereby preventing its response to viral infection. In addition, BDCA-2 plays a crucial role in autoimmune diseases. Elevated IFN-α levels can sometimes be detected in autoimmune diseases such as systemic lupus erythematosus (SLE), which may be caused by the overactivation of pDCs. As a suppressor of IFN-α production, BDCA-2 may help prevent excessive autoimmune reactions, thereby avoiding the development of autoimmune diseases. In summary, BDCA-2 plays a key role in regulating pDC responses, resisting viral infections, and preventing certain autoimmune diseases. Current research continues to explore the functions and clinical application prospects of BDCA-2.

### Summary

In view of the above shortcomings, the present invention provides an anti-BDCA-2 antibody, preparation method therefor and use thereof. This antibody and related drugs can be used for inhibiting the generation of IFN-alpha by pDCs and prevent overactivation of pDCs. The anti-BDCA-2 antibody can effectively inhibit the activation of pDC cells and the production of IFN-α, and has great potential in resisting virus infection and preventing certain autoimmune diseases.

### Interpretation of terms:

Unless otherwise defined herein, scientific and technical terms related to this document shall have the meanings as understood by ordinary people in this field. Generally, the nomenclature and techniques related to pharmacology, biology, biochemistry, cell and tissue culture, biology, molecular biology, immunology, microbiology, genetics, and protein and nucleic acid chemistry, as well as hybridization, described herein are well-known and frequently used in the field.

In this invention, " BDCA-2" refers to blood dendritic cell antigen. The term includes variants, homologs, orthologs, and paralogs. For example, antibodies specific to human BDCA-2 can cross-react with the BDCA-2 protein of another species, such as monkeys, under certain circumstances. In other implementations, antibodies specific to human BDCA-2 protein can be completely specific to human BDCA-2 protein without cross-reactivity with proteins of other species or other types, or can cross-react with BDCA-2 protein of some other species but not all other species.

In this invention, "amino acid" includes natural amino acids, synthetic amino acids, and amino acid analogs and amino acid mimics that function in a manner similar to that of natural amino acids. Natural amino acids are amino acids encoded by the genetic code. Amino acid analogs are those that have the same basic chemical structure as naturally occurring amino acids. Amino acids may be mentioned herein by their commonly known three-letter symbols or by the single-letter symbols recommended by the IUPAC-IUB Committee on Biochemical Nomenclature.

In this invention, the "complementarity-determining region" is also referred to as the CDR. The complementarity-determining region disclosed in this article refers to the antigen-binding site within the variable region of the heavy or light chain of the antibody, i.e., the amino acid residue in the antibody that causes antigen binding. The complementarity-determining region of the light and heavy chains together form the antigen-binding site of the antibody. In general, the light or heavy chain of an antibody typically contains three determinant complementary regions. In this article, when the complementarity-determining region refers to the complementarity-determining region of the heavy chain, it is abbreviated as HCDR, and three different complementarity-determining regions are referred to by numbers, such as HCDR1, HCDR2, and HCDR3. In this article, when the complementarity-determining region refers to the complementarity-determining region of the light chain, it is abbreviated as LCDR, and three different complementarity-determining regions are referred to by numbers, such as LCDR1, LCDR2, and LCDR3.

In this invention, "antigen-binding portion", "antigen-binding domain", "antigen-binding region" or "antigen-binding site" refers to a portion of an antibody that contains amino acid residues that interact with the antigen and contribute to the specificity and affinity of the antibody to the antigen. For antibodies that specifically bind to their antigens, this will include at least a portion or at least one of their CDR domains.

In this invention, "the variable region" refers to the region of the immunoglobulin light chain and heavy chain near the N-terminus where the amino acid sequence changes significantly.

In this invention, "the heavy chain" refers to two identical heavy chains (H chains) with larger relative molecular weight in the antibody, "the light chain" refers to two shorter identical light chains (L chains) with smaller relative molecular weight molecular weight in the antibody.

In this invention, "similarity" refers to the consistency of the amino acid sequence of homologous proteins and the proportion of substituted amino acids.

In this invention, "monoclonal antibody" refers to an antibody molecule product composed of a single molecule. Monoclonal antibodies exhibit single binding specificity and affinity for specific epitopes. In this invention, murine antibodies refer to antibodies secreted by murine hybrid fusion cells obtained by fusing B cells from immunized mice with myeloma cells.

In this invention, the term "EC₅₀" is also called the half-maximal effect concentration, which refers to the antibody concentration that causes a 50% maximum effect.

In this invention, the term "EC₅₀" is also called half-inhibitory concentration. The IC₅₀ value can be used to measure the ability of a drug to induce apoptosis. That is, the stronger the induction ability, the lower the value. It can also conversely indicate the degree of tolerance of a certain cell to a drug.

In one aspect, the present invention provides a preparation method for anti-BDCA-2 antibody, including following steps:
(1) fusing mouse B cells and myeloma cells to obtain hybridoma cells;
(2) ELISA detecting of culture supernatant of the hybridoma cells and antigen protein to obtain positive clones;
(3) subcloning the positive clones and screening to obtain a monoclonal antibody;
(4) encoding an antibody nucleotide sequence and constructing an expression vector;
(5) transfecting host cells and obtaining an anti-BDCA-2 antibody through host cell expression.

In another aspect, the present invention provides an anti-BDCA-2 antibody, including a light chain and a heavy chain, the light chain includes a light chain complementarity-determining region and the heavy chain includes a heavy chain complementarity-determining region;
or, the light chain complementarity-determining region includes LCDR1, LCDR2, and LCDR3 as shown in SEQ ID NO: 25, GAT, and SEQ ID NO: 26, the heavy chain complementarity-determining region includes HCDR1, HCDR2, and HCDR3 as shown in SEQ ID NO: 27, SEQ ID NO: 28, and SEQ ID NO: 29;
or, the light chain complementarity-determining region includes LCDR1, LCDR2, and LCDR3 as shown in SEQ ID NO: 30, LAS, and SEQ ID NO: 31, the heavy chain complementarity-determining region includes HCDR1, HCDR2, and HCDR3 as shown in SEQ ID NO: 32, SEQ ID NO: 33, and SEQ ID NO: 34;
or, the light chain complementarity-determining region includes LCDR1, LCDR2, and LCDR3 as shown in SEQ ID NO: 35, WAS, and SEQ ID NO: 36, the heavy chain complementarity-determining region includes HCDR1, HCDR2, and HCDR3 as shown in SEQ ID NO: 37, SEQ ID NO: 38, and SEQ ID NO: 39;
or, the light chain complementarity-determining region includes LCDR1, LCDR2, and LCDR3 as shown in SEQ ID NO: 40, RAS, and SEQ ID NO: 41, the heavy chain complementarity-determining region includes HCDR1, HCDR2, and HCDR3 as shown in SEQ ID NO: 42, SEQ ID NO: 43, and SEQ ID NO: 44;
or, the light chain complementarity-determining region includes LCDR1, LCDR2, and LCDR3 as shown in SEQ ID NO: 45, SAS, and SEQ ID NO: 46, the heavy chain complementarity-determining region includes HCDR1, HCDR2, and HCDR3 as shown in SEQ ID NO: 47, SEQ ID NO: 48, and SEQ ID NO: 49;
or, the light chain complementarity-determining region includes LCDR1, LCDR2, and LCDR3 as shown in SEQ ID NO: 50, AAS, and SEQ ID NO: 51, the heavy chain complementarity-determining region includes HCDR1, HCDR2, and HCDR3 as shown in SEQ ID NO: 52, SEQ ID NO: 53, and SEQ ID NO: 54;
or, the light chain complementarity-determining region includes LCDR1, LCDR2, and LCDR3 as shown in SEQ ID NO: 55, WAS, and SEQ ID NO: 56, the heavy chain complementarity-determining region includes HCDR1, HCDR2, and HCDR3 as shown in SEQ ID NO: 57, SEQ ID NO: 58, and SEQ ID NO: 59;
or, the light chain complementarity-determining region includes LCDR1, LCDR2, and LCDR3 as shown in SEQ ID NO: 60, YSS, and SEQ ID NO: 61, the heavy chain complementarity-determining region includes HCDR1, HCDR2, and HCDR3 as shown in SEQ ID NO: 62, SEQ ID NO: 63, and SEQ ID NO: 64;
or, the light chain complementarity-determining region includes LCDR1, LCDR2, and LCDR3 as shown in SEQ ID NO: 65, SAS, and SEQ ID NO: 66, the heavy chain complementarity-determining region includes HCDR1, HCDR2, and HCDR3 as shown in SEQ ID NO: 67, SEQ ID NO: 68, and SEQ ID NO: 69;
or, the light chain complementarity-determining region includes LCDR1, LCDR2, and LCDR3 as shown in SEQ ID NO: 70, RAS, and SEQ ID NO: 71, the heavy chain complementarity-determining region includes HCDR1, HCDR2, and HCDR3 as shown in SEQ ID NO: 72, SEQ ID NO: 73, and SEQ ID NO: 74;
or, the light chain complementarity-determining region includes LCDR1, LCDR2, and LCDR3 as shown in SEQ ID NO: 75, GTS, and SEQ ID NO: 76, the heavy chain complementarity-determining region includes HCDR1, HCDR2, and HCDR3 as shown in SEQ ID NO: 77, SEQ ID NO: 78, and SEQ ID NO: 79.

Specifically, a light chain variable region of the antibody includes any one of amino acid sequences shown in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, and SEQ ID NO: 21.

Specifically, a heavy chain variable region of the antibody includes any one of amino acid sequences shown in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, and SEQ ID NO: 22.

Specifically, an amino acid sequence of a light chain variable region of the antibody is shown in SEQ ID NO: 1, an amino acid sequence of a heavy chain variable region of the antibody is shown in SEQ ID NO: 2;
or, an amino acid sequence of a light chain variable region of the antibody is shown in SEQ ID NO: 3, an amino acid sequence of a heavy chain variable region of the antibody is shown in SEQ ID NO: 4;
or, an amino acid sequence of a light chain variable region of the antibody is shown in SEQ ID NO: 5, an amino acid sequence of a heavy chain variable region of the antibody is shown in SEQ ID NO: 6;
or, an amino acid sequence of a light chain variable region of the antibody is shown in SEQ ID NO: 7, an amino acid sequence of a heavy chain variable region of the antibody is shown in SEQ ID NO: 8;
or, an amino acid sequence of a light chain variable region of the antibody is shown in SEQ ID NO: 9, an amino acid sequence of a heavy chain variable region of the antibody is shown in SEQ ID NO: 10;
or, an amino acid sequence of a light chain variable region of the antibody is shown in SEQ ID NO: 11, an amino acid sequence of a heavy chain variable region of the antibody is shown in SEQ ID NO: 12;
or, an amino acid sequence of a light chain variable region of the antibody is shown in SEQ ID NO: 13, an amino acid sequence of a heavy chain variable region of the antibody is shown in SEQ ID NO: 14;
or, an amino acid sequence of a light chain variable region of the antibody is shown in SEQ ID NO: 15, an amino acid sequence of a heavy chain variable region of the antibody is shown in SEQ ID NO: 16;
or, an amino acid sequence of a light chain variable region of the antibody is shown in SEQ ID NO: 17, an amino acid sequence of a heavy chain variable region of the antibody is shown in SEQ ID NO: 18;
or, an amino acid sequence of a light chain variable region of the antibody is shown in SEQ ID NO: 19, an amino acid sequence of a heavy chain variable region of the antibody is shown in SEQ ID NO: 20.
or, an amino acid sequence of a light chain variable region of the antibody is shown in SEQ ID NO: 21, an amino acid sequence of a heavy chain variable region of the antibody is shown in SEQ ID NO: 22.

Specifically, the light chain is formed by splicing a light chain variable region and a light chain constant region, and the heavy chain is formed by splicing a heavy chain variable region and a heavy chain constant region.

More specifically, the variable region of the light chain includes a κ constant region.

Preferably, the light chain constant region includes the amino acid sequence shown in SEQ ID NO:80.

More specifically, the heavy chain variable region includes IgG1, IgG2, IgG3, IgG4 or IgG1-YTE heavy chain constant region.

Preferably, the heavy chain variable region includes the amino acid sequence shown in SEQ ID NO:81.

Specifically, the amino acid sequence of the light chain variable region of the antibody is shown in SEQ ID NO: 82, the amino acid sequence of the heavy chain variable region of the antibody is shown in SEQ ID NO: 83;
or, the amino acid sequence of the light chain of the antibody is shown in SEQ ID NO: 84, the amino acid sequence of the heavy chain of the antibody is shown in SEQ ID NO: 85;
or, the amino acid sequence of the light chain of the antibody is shown in SEQ ID NO: 86, the amino acid sequence of the heavy chain of the antibody is shown in SEQ ID NO: 87;
or, the amino acid sequence of the light chain of the antibody is shown in SEQ ID NO: 88, the amino acid sequence of the heavy chain of the antibody is shown in SEQ ID NO: 89;
or, the amino acid sequence of the light chain of the antibody is shown in SEQ ID NO: 90, the amino acid sequence of the heavy chain of the antibody is shown in SEQ ID NO: 91;
or, the amino acid sequence of the light chain of the antibody is shown in SEQ ID NO: 92, the amino acid sequence of the heavy chain of the antibody is shown in SEQ ID NO: 93;
or, the amino acid sequence of the light chain of the antibody is shown in SEQ ID NO: 94, the amino acid sequence of the heavy chain of the antibody is shown in SEQ ID NO: 95;
or, the amino acid sequence of the light chain of the antibody is shown in SEQ ID NO: 96, the amino acid sequence of the heavy chain of the antibody is shown in SEQ ID NO: 97;
or, the amino acid sequence of the light chain of the antibody is shown in SEQ ID NO: 98, the amino acid sequence of the heavy chain of the antibody is shown in SEQ ID NO: 99;
or, the amino acid sequence of the light chain of the antibody is shown in SEQ ID NO: 100, the amino acid sequence of the heavy chain of the antibody is shown in SEQ ID NO: 101.
or, the amino acid sequence of the light chain of the antibody is shown in SEQ ID NO: 102, the amino acid sequence of the heavy chain of the antibody is shown in SEQ ID NO: 103.

In another aspect, the present invention provides a nucleic acid which encodes the above-mentioned antibody.

In another aspect, the present invention provided an expression vector encoding the above-mentioned nucleic acid.

In another aspect, the present invention provides a host cell including the above-mentioned expression vector.

In another aspect, the present invention provides a use of the aforementioned antibody in drug preparation.

Specifically, the drug promotes BDCA-2 endocytosis, inhibits excessive activation of pDC, or inhibits pDC from generation of IFN-α by binding the anti-BDCA-2 antibody with BDCA-2.

In another aspect, the present invention provides a pharmaceutical composition including the above-mentioned anti-BDCA-2 antibody, nucleic acid, expression vector, and/or host cell.

Specifically, the pharmaceutical composition further includes at least one of a pharmaceutically acceptable carrier or a pharmaceutically acceptable auxiliary material.

### The beneficial technical effects of the present invention are in that:

(1) The anti-BDCA-2 antibody and related drugs can be used for inhibiting the production of IFN-α by pDCs and preventing the over-activation of the pDCs.
(2) The anti-BDCA-2 antibody can effectively inhibit the activation of pDC cells and the production of IFN-α, and has great potential in resisting virus infection and preventing certain autoimmune diseases.

### BRIEF DESCRIPTION OF THE DRAWINGS

FIG. 1 shows the binding of anti-BDCA-2 antibody to human BDCA-2.
FIG. 2 shows the binding of anti-BDCA-2 antibody to monkey BDCA-2.
FIG. 3 shows the binding of anti-BDCA-2 antibody to RPMI8226-hBDCA2 cells.
FIG. 4 shows the endocytosis detection of anti-BDCA-2 antibody.
FIG. 5 shows that the anti-BDCA-2 antibody inhibits the secretion of IFN-α in PBMC (Donor1) stimulated by TLR-ligand.
FIG. 6 shows that the anti-BDCA-2 antibody inhibits the secretion of IFN-α in PBMC (Donor2) stimulated by TLR-ligand.
FIG. 7 shows that the anti-BDCA-2 antibody inhibits the secretion of IFN-α in PBMC (Donor2) stimulated by TLR-ligand.

### DETAILED DESCRIPTION

The invention will be described below with specific examples, which are not used to limit the invention, but only to illustrate the invention, so as to make the technical scheme of the invention easier to understand and master. Unless otherwise specified, the experimental methods described in the following embodiments are conventional methods; the reagents and materials described are commercially available unless otherwise specified.

**The instruments used in this invention are as follows:**
1. ELISA reader: Instrument manufacturer: Thermo Fisher; Instrument model: Multiskan FC.
2. Flow cytometer: Manufacturer: Coulter Beckman; Model: CytoFLEX S.
3. High-content imaging: Manufacturer: PerkinElmer; Instrument model: Operetta CLS.

### Basic Experiment Example 1: Fusion of Mouse Immunity and Hybridoma

An equal volume of PBS containing 50µg antigen and emulsion of Freund's complete adjuvant (200µL/mouse) were prepared: syringes containing antigen and adjuvant were connected respectively using a three-way valve. The syringe was pushed back and forth to move its contents from one side of the syringe to the other, and continued pushing for 10 minutes until a stable emulsion was obtained. The emulsified solution was injected subcutaneously at multiple points on the back of 6-8 weeks old Balb/c mice (purchased from Beijing Charles River Laboratory Animal Technology Co., Ltd., and procured and bred by Suzhou GenePharma Co., Ltd.) using a syringe, and a total of 5 mice were immunized. Two weeks later, an equal volume of PBS containing 25µg antigen and emulsion prepared from incomplete Freund's adjuvant (200µL) was injected subcutaneously at multiple sites. Two weeks later, the mice were given a booster immunization, and blood titers were measured 7 days after the booster immunization. When the antibody titer is high enough (≥1/50000), booster immunization was administered via subcutaneous tail vein injection of PBS solution containing 25 µg of antigen 3 days before fusion. Three days after booster immunization, spleens were collected from two mice after dissection. Spleen lymphocytes were isolated from the mice and fused with SP2/0 cells for hybridoma fusion.

### Example 1 Molecular screening of binding BDCA-2 protein

Recombinant human BDCA-2 (purchased from Suzhou Novoprotein Technology Co., Ltd., NP: PLKAA-1, LOT: 20220816) and recombinant monkey BDCA-2 (purchased from Suzhou Novoprotein Technology Co., Ltd., NP: PLRBB, LOT: 20211220) were used as antigens to coat high-affinity ELISA plates overnight at 4°C, with a coating amount of 50 ng per well. The plates were then blocked with 2% BSA at 37°C for 2 h. 100 µL of hybridoma supernatant was incubated with recombinant human BDCA-2 and recombinant monkey BDCA-2 for 1 h respectively, and then the plates were washed 3 times with washing solution. freshly diluted enzyme-labeled antibody was added, incubated at 37°C for 30 minutes, and then washed. 100 µL of TMB substrate solution was added, the color was developed for 5-10 minutes, then 50 µL of 2M sulfuric acid was added to terminate the reaction. The absorbance at 450 nm was read using an ELISA reader. If the reading was 2.1 times greater than the OD value of the negative control, it was considered a clone that was positive binding to recombinant human BDCA-2 or recombinant monkey BDCA-2.

Fresh hybridoma cell precipitates were collected from positive binding clones, the number of which was more than 5 × 10⁶, and transported on dry ice to Suzhou Genewiz Biotechnology Co., Ltd. for monoclonal antibody sequencing after Flash-frozen in liquid nitrogen.Molecules with complete variable region sequences were obtained, as shown in Table 1 below.

**Table 1 Different Cloning Sequences**

| Antib odies | Light chain variable region | Light chain CDR sequence | | | Heavy chain variable region | Heavy chain CDR sequence | | |
|---|---|---|---|---|---|---|---|---|
| | | LCDR1 | LCDR2 | LCDR3 | | HCDR1 | HCDR2 | HCDR3 |
| 12D5 B12 | SEQ ID NO: 1 | SEQ ID NO: 25 | GAT | SEQ ID NO: 26 | SEQ ID NO: 2 | SEQ ID NO: 27 | SEQ ID NO: 28 | SEQ ID NO: 29 |
| 1C11 F3 | SEQ ID NO: 3 | SEQ ID NO: 30 | LAS | SEQ ID NO: 31 | SEQ ID NO: 4 | SEQ ID NO: 32 | SEQ ID NO: 33 | SEQ ID NO: 34 |
| 4H6B 5 | SEQ ID NO: 5 | SEQ ID NO: 35 | WAS | SEQ ID NO: 36 | SEQ ID NO: 6 | SEQ ID NO: 37 | SEQ ID NO: 38 | SEQ ID NO: 39 |
| 9H6C 7 | SEQ ID NO: 7 | SEQ ID NO: 40 | RAS | SEQ ID NO: 41 | SEQ ID NO: 8 | SEQ ID NO: 42 | SEQ ID NO: 43 | SEQ ID NO: 44 |
| 20B2 C1 | SEQ ID NO: 9 | SEQ ID NO: 45 | SAS | SEQ ID NO: 46 | SEQ ID NO: 10 | SEQ ID NO: 47 | SEQ ID NO: 48 | SEQ ID NO: 49 |
| 4A2C 12 | SEQ ID NO: 11 | SEQ ID NO: 50 | AAS | SEQ ID NO: 51 | SEQ ID NO: 12 | SEQ ID NO: 52 | SEQ ID NO: 53 | SEQ ID NO: 54 |
| 14E6 A12 | SEQ ID NO: 13 | SEQ ID NO: 55 | WAS | SEQ ID NO: 56 | SEQ ID NO: 14 | SEQ ID NO: 57 | SEQ ID NO: 58 | SEQ ID NO: 59 |
| 4B3F 5 | SEQ ID NO: 15 | SEQ ID NO: 60 | YSS | SEQ ID NO: 61 | SEQ ID NO: 16 | SEQ ID NO: 62 | SEQ ID NO: 63 | SEQ ID NO: 64 |
| 5H6F 9 | SEQ ID NO: 17 | SEQ ID NO: 65 | SAS | SEQ ID NO: 66 | SEQ ID NO: 18 | SEQ ID NO: 67 | SEQ ID NO: 68 | SEQ ID NO: 69 |
| 6G5H 6 | SEQ ID NO: 19 | SEQ ID NO: 70 | RAS | SEQ ID NO: 71 | SEQ ID NO: 20 | SEQ ID NO: 72 | SEQ ID NO: 73 | SEQ ID NO: 74 |
| 11G9 B3 | SEQ ID NO: 21 | SEQ ID NO: 75 | GTS | SEQ ID NO: 76 | SEQ ID NO: 22 | SEQ ID NO: 77 | SEQ ID NO: 78 | SEQ ID NO: 79 |

### Example 2 Construction and expression of anti-BDCA-2 antibody

The light chain variable region and light chain constant region (SEQ ID NO:80) of the sequenced monoclonal antibody were directly spliced together to form the light chain, and the heavy chain variable region and heavy chain constant region (SEQ ID NO:81) of the sequenced monoclonal antibody were directly spliced together to form the heavy chain. The amino acid sequences of the light chain and the heavy chain of the antibody are shown in SEQ ID NO: 82 and SEQ ID NO: 83;
or, the amino acid sequences shown in SEQ ID NO:84 and SEQ ID NO:85;
or, the amino acid sequences shown in SEQ ID NO:86 and SEQ ID NO:87;
or, the amino acid sequences shown in SEQ ID NO:88 and SEQ ID NO:89;
or, the amino acid sequences shown in SEQ ID NO:90 and SEQ ID NO:91;
or, the amino acid sequences shown in SEQ ID NO:92 and SEQ ID NO:93;
or, the amino acid sequences shown in SEQ ID NO:94 and SEQ ID NO:95;
or, the amino acid sequences shown in SEQ ID NO:96 and SEQ ID NO:97;
or, the amino acid sequences shown in SEQ ID NO:98 and SEQ ID NO:99;
or, the amino acid sequences shown in SEQ ID NO:100 and SEQ ID NO:101;
or, the amino acid sequences shown in SEQ ID NO:102 and SEQ ID NO:103;

For the amino acid sequences of the light and heavy chains, codon optimization was performed according to human host cells, and the gene was synthesized conventionally (the amino acid sequences of the light chain constant region and the heavy chain constant region are shown in SEQ ID NO: 80 and SEQ ID NO: 81, respectively). The gene was cloned into the pTT5 vector (ampicillin resistance) using 5'EcoRI and 3'HindIII. Clones were selected for sequencing. The cells with correct sequencing results were selected for preservation and amplification culture. The amplified cells were then used for plasmid extraction.
The bacteria with correct sequencing was selected to preserve species and expanded culture of bacteria was performed. The expanded bacteria were used for plasmid extraction. Following the same gene synthesis and vector construction methods described above, a plasmid expressing the control antibody was obtained. In particular, the heavy chain of the control antibody is shown in SEQ ID NO: 109; and the light chain of the control antibody is shown in SEQ ID NO: 110.

**Table 2 Control antibody sequences**

| Antib odies | Light chain variable region | Light chain CDR sequence | | | Heavy chain variable region | Heavy chain CDR sequence | | |
|---|---|---|---|---|---|---|---|---|
| | | LCDR1 | LCDR2 | LCDR3 | | HCDR1 | HCDR2 | HCDR3 |
| PC | SEQ ID NO: 23 | SEQ ID NO: 104 | AAS | SEQ ID NO: 105 | SEQ ID NO: 24 | SEQ ID NO: 106 | SEQ ID NO: 107 | SEQ ID NO: 108 |

The extracted plasmids were transfected into cells and the proteins were isolated and purified as follows:
1. HEK-293F cells were cultured in Free Style^{™} 293 expression medium (Gibco, Cat#: 12338-018). Cell density was measured, and the viability was greater than 95%. Cells were collected by centrifugation, and the cell density was adjusted to 5 × 10⁶ cells/mL using a cell counter.
2. 10% MEM of transfection system was added into 50mL centrifuge tube, and plasmid was added, mixed well, filtered, and stand for 5min, PEI was added to the DNA suspension (the ratio of DNA to PEI is 1:3, and the amount of DNA added per milliliter of cell culture medium is 1.5µg), mixed gently (invert gently 2-3 times to mix), and stand for 15-20min. The complex was then gently added to the dispensed cells while gently shaking the flask. The transfected HEK-293F cells were then placed in a 37°C, 5% CO₂ incubator and cultured at 120 rpm. After culturing for 10-12 days, the supernatant was collected.
3. Anti-BDCA-2 antibody was enriched and purified by a pre-equilibrated protein-G affinity column (GE, Cat#: 17040501) and eluted with elution buffer (20 mM citric acid, pH 3.0-pH 3.5). The anti-BDCA-2 antibody was then placed in PBS at pH 7.0, and the antibody concentration was detected by NanoDrop.

### Example 3: Cross-binding of anti-BDCA-2 antibody with human and monkey BDCA-2.

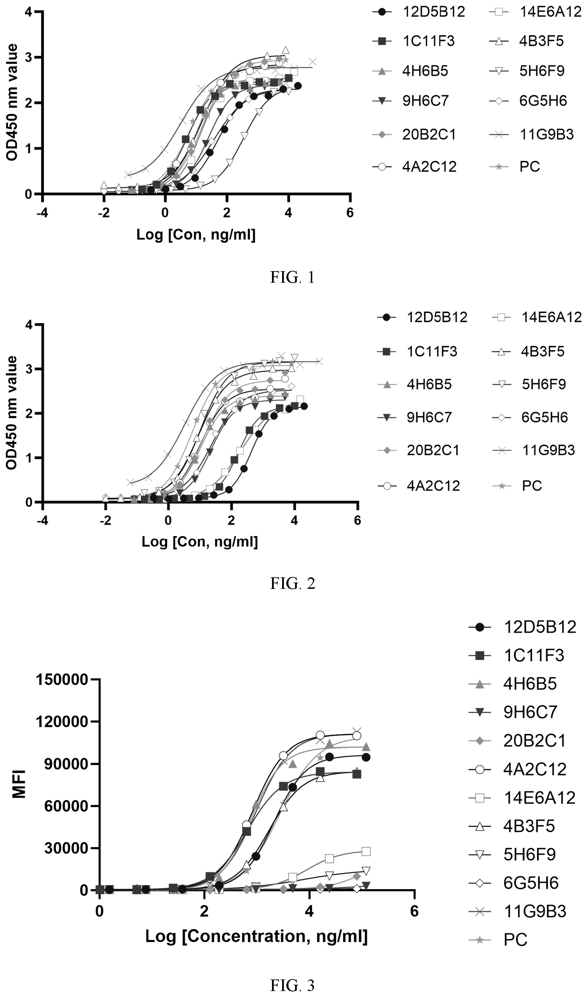

In order to determine whether the anti-BDCA-2 antibody cross-binds with human and monkey BDCA-2, ELISA was performed using recombinant human and monkey BDCA-2 proteins. The specific steps were as follows:
Recombinant human BDCA-2 and recombinant monkey BDCA-2 were used as antigens to coat high-affinity ELISA plates overnight at 4°C, with a coating amount of 50 ng per well. The plates were then blocked with 2% BSA at 37°C for 2 h. Each serially 100µL diluted antibody with different concentration was incubated with recombinant human BDCA-2 and recombinant monkey BDCA-2 for 1 h, and then the plates were washed three times with washing buffer. freshly diluted enzyme-labeled antibody was added, incubated at 37°C for 30 minutes, and then washed. 100 µL of TMB substrate solution was added, the color was developed for 5-10 minutes, then 50 µL of 2M sulfuric acid was added to terminate the reaction. The absorbance at 450 nm was read using an ELISA reader, and the binding curve was plotted as shown in FIG. 1 and FIG. 2. The EC₅₀ values were calculated, and the EC₅₀ values for each antibody are shown in Table 3.

**Table 3 Anti-BDCA-2 antibody binding to human and monkey BDCA-2 protein**

| Antibodies | EC₅₀ (ng/ml) | |
|---|---|---|
| | Human BDCA-2 | Monkey BDCA-2 |
| 12D5B12 | 49.92 | 413.2 |
| 1C11F3 | 5.126 | 161.3 |
| 4H6B5 | 8.787 | 10.26 |
| 9H6C7 | 24.91 | 22.36 |
| 20B2C1 | 16.06 | 12.19 |
| 4A2C12 | 11.74 | 10.27 |
| 14E6A12 | 13.57 | 202.4 |
| 4B3F5 | 15.34 | 9.148 |
| 5H6F9 | 343.5 | 10.39 |
| 6G5H6 | 36.36 | 23.74 |
| 11G9B3 | 3.099 | 3.295 |
| PC | 6.559 | 5.61 |

### Example 4: Anti-BDCA-2 antibody binds to human BDCA-2

In order to determine whether the anti-BDCA-2 antibody binds to human BDCA-2 expressed on the cell surface, FACS cell binding assays were performed using RPMI8226 cells stably overexpressing human BDCA-2 (purchased from Fuheng Biology, catalog number FH0092).

10⁵ RPMI8226 cells were seeded in 100 µL of culture medium onto a 96-well V plate, and 50 µL of serially diluted anti-BDCA-2 antibody of each concentration was added. After incubation at 4°C for 1 hour, the 96-well plate was washed three times with PBST. 1000 times diluted PE- goat anti-human IgG (purchased from BioLegend, catalog number 366904) was added. After incubation at 4°C for 1 hour, the 96-well plate was washed three times with PBS, and cell fluorescence was detected by flow cytometry. The binding curve was plotted as shown in FIG. 3. The EC₅₀ values were calculated, and the EC₅₀ values for each antibody are shown in Table 4.

**Table 4 Anti-BDCA-2 antibody binds to human BDCA-2**

| Antibodies | EC₅₀ (ng/ml) |
|---|---|
| 12D5B12 | 2106 |
| 1C11F3 | 653.3 |
| 4H6B5 | 770.9 |
| 9H6C7 | Weak binding |
| 20B2C1 | Weak binding |
| 4A2C12 | 819.7 |
| 14E6A12 | 8790 |
| 4B3F5 | 1693 |
| 5H6F9 | 7483 |
| 6G5H6 | Weak binding |
| 11G9B3 | 955.4 |
| PC | 2741 |

### Example 5: Detection of endocytosis of anti-BDCA-2 antibody

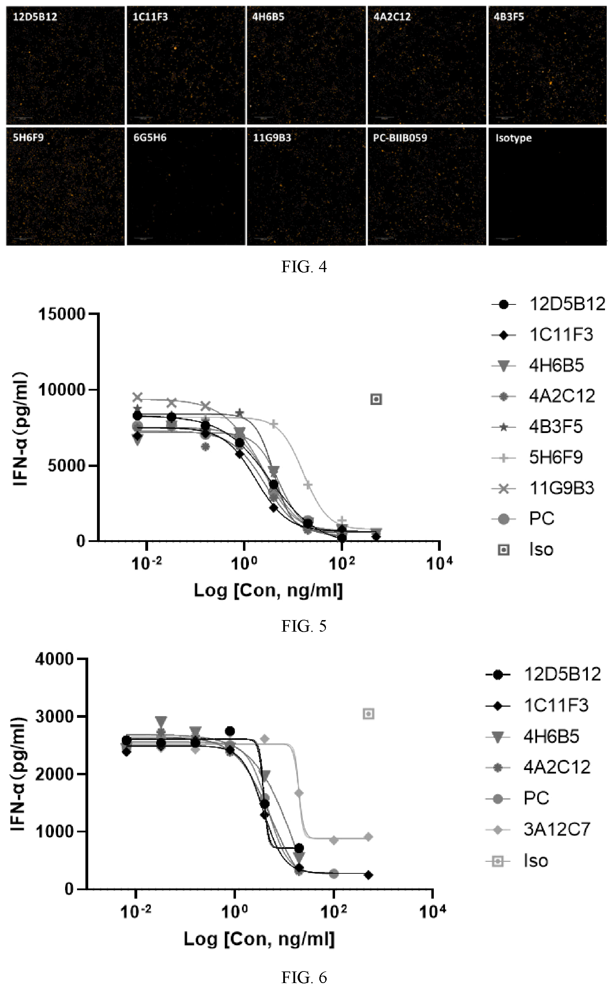

RPMI8226 cells stably overexpressing human BDCA-2 were used for endocytosis detection by FACS and high-content imaging. The antibody to be tested was labeled with Zenon^{™} pHrodo^{™} iFL Green Human IgG Labeling Reagent, and then RPMI8226-hBDCA2 cells and the labeled antibody were incubated at 37°C for 16 hours. The cells were then collected and washed. The image was taken using high-content imaging, and the result is shown in FIG. 4. Fluorescence intensity was detected by flow cytometry, and the results are shown in Table 5.

**Table 5 Endocytotic flow cytometry detection of BDCA-2 antibodies**

| Antibodies | MFI |
|---|---|
| 12D5B12 | 2619 |
| 1C11F3 | 2405 |
| 4H6B5 | 1863 |
| 9H6C7 | 699 |
| 20B2C1 | 721 |
| 4A2C12 | 2352 |
| 14E6A12 | 1785 |
| 4B3F5 | 2645 |
| 5H6F9 | 3414 |
| 6G5H6 | 397 |
| 11G9B3 | 2735 |
| PC | 2272 |
| iRed | 224 |
| Blank | 73.7 |
| Isotype | 256 |

Antibodies 12D5B12, 1C11F3, 4H6B5, 9H6C7, 20B2C1, 4A2C12, 14E6A12, 4B3F5, 5H6F9, and 11G9B3 showed obvious red fluorescence. Compared to the control group, the above anti-BDCA-2 antibodies exhibit significant endocytosis activity.

### Example 6: Anti-BDCA-2 antibody inhibits TLR-ligand-stimulated PBMC IFN-α secretion.

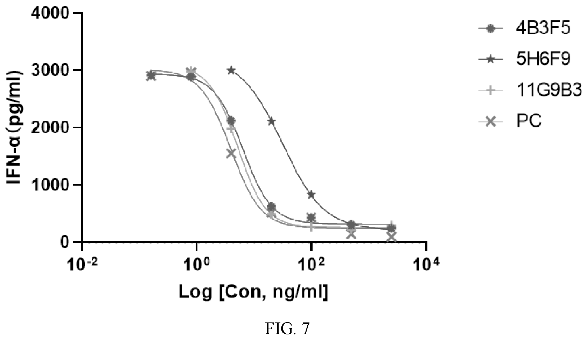

In this embodiment, human peripheral blood lymphocytes (PBMCs, purchased from Shanghai Sailybio technology Co., Ltd., catalog number XFB-HP050B) were used as the research object, and ODN2216 was used to stimulate PBMCs to secrete IFN-α. Simultaneously with ODN2216 stimulation, a 5-fold serially diluted anti-BDCA-2 antibody was added to the coated cells. Cells containing ODN2216 and different concentrations of the test antibody were incubated overnight (18 hours) in a 37°C cell culture incubator. Cell supernatant was collected and the IFN-α content in the supernatant was detected using a commercially available Human IFN-α ELISA Kit (purchased from absin, catalog number abs51025-96T). The inhibition curve was depicted, and the result is shown in FIGs. 5-7. The IC₅₀ values were calculated, and the IC50 values for each antibody are shown in Table 6.

**Table 6 IC₅₀ values of anti-BDCA-2 antibodies**

| Antibodies | IC₅₀ (ng/ml) | |
|---|---|---|
| | Donor 1 | Donor 2 |
| 12D5B12 | 3.432 | 3.863 |
| 1C11F3 | 1.728 | 3.727 |
| 4H6B5 | 5.146 | 20.23 |
| 4A2C12 | 2.644 | 5.357 |
| 4B3F5 | 4.04 | 6.376 |
| 5H6F9 | 16.38 | 31.22 |
| 11G9B3 | 2.462 | 5.151 |
| PC | 3.012 | 4.615 |

The above results indicate that: the anti-BDCA-2 antibody of the present invention can effectively inhibit the activation of pDC cells and the generation of IFN-α.

The above detailed description is a specific description of one feasible embodiment of the present invention. The embodiment is not intended to limit the patent scope of the present invention. It should be pointed out that, any equivalent implementation or modification that does not deviate from the present invention should be included in the scope of the technical solution of the present invention. Therefore, the scope of protection of this invention shall be determined by the appended claims.

## Claims

1. A preparation method for anti-BDCA-2 antibody, **characterized in that**, comprising following steps:
(1) fusing mouse B cells and myeloma cells to obtain hybridoma cells;
(2) ELISA detecting of culture supernatant of the hybridoma cells with antigen protein to obtain positive clones;
(3) subcloning the positive clones and screening to obtain a monoclonal antibody;
(4) encoding an antibody nucleotide sequence and constructing an expression vector;
(5) transfecting host cells and obtaining an anti-BDCA-2 antibody through host cell expression.

2. An anti-BDCA-2 antibody, **characterized in that**, the antibody comprises a light chain and a heavy chain, wherein the light chain comprises a light chain complementarity-determining region, and the heavy chain comprises a heavy chain complementarity-determining region, the light chain complementarity-determining region comprises LCDR1, LCDR2, and LCDR3 as shown in SEQ ID NO: 25, GAT, and SEQ ID NO: 26, the heavy chain complementarity-determining region comprises HCDR1, HCDR2, and HCDR3 as shown in SEQ ID NO: 27, SEQ ID NO: 28, and SEQ ID NO: 29;
or, the light chain complementarity-determining region comprises LCDR1, LCDR2, and LCDR3 as shown in SEQ ID NO: 30, LAS, and SEQ ID NO: 31, the heavy chain complementarity-determining region comprises HCDR1, HCDR2, and HCDR3 as shown in SEQ ID NO: 32, SEQ ID NO: 33, and SEQ ID NO: 34;
or, the light chain complementarity-determining region comprises LCDR1, LCDR2, and LCDR3 as shown in SEQ ID NO: 35, WAS, and SEQ ID NO: 36, the heavy chain complementarity-determining region comprises HCDR1, HCDR2, and HCDR3 as shown in SEQ ID NO: 37, SEQ ID NO: 38, and SEQ ID NO: 39;
or, the light chain complementarity-determining region comprises LCDR1, LCDR2, and LCDR3 as shown in SEQ ID NO: 40, RAS, and SEQ ID NO: 41, the heavy chain complementarity-determining region comprises HCDR1, HCDR2, and HCDR3 as shown in SEQ ID NO: 42, SEQ ID NO: 43, and SEQ ID NO: 44;
or, the light chain complementarity-determining region comprises LCDR1, LCDR2, and LCDR3 as shown in SEQ ID NO: 45, SAS, and SEQ ID NO: 46, the heavy chain complementarity-determining region comprises HCDR1, HCDR2, and HCDR3 as shown in SEQ ID NO: 47, SEQ ID NO: 48, and SEQ ID NO: 49;
or, the light chain complementarity-determining region comprises LCDR1, LCDR2, and LCDR3 as shown in SEQ ID NO: 50, AAS, and SEQ ID NO: 51, the heavy chain complementarity-determining region comprises HCDR1, HCDR2, and HCDR3 as shown in SEQ ID NO: 52, SEQ ID NO: 53, and SEQ ID NO: 54;
or, the light chain complementarity-determining region comprises LCDR1, LCDR2, and LCDR3 as shown in SEQ ID NO: 55, WAS, and SEQ ID NO: 56, the heavy chain complementarity-determining region comprises HCDR1, HCDR2, and HCDR3 as shown in SEQ ID NO: 57, SEQ ID NO: 58, and SEQ ID NO: 59;
or, the light chain complementarity-determining region comprises LCDR1, LCDR2, and LCDR3 as shown in SEQ ID NO: 60, YSS, and SEQ ID NO: 61, the heavy chain complementarity-determining region comprises HCDR1, HCDR2, and HCDR3 as shown in SEQ ID NO: 62, SEQ ID NO: 63, and SEQ ID NO: 64;
or, the light chain complementarity-determining region comprises LCDR1, LCDR2, and LCDR3 as shown in SEQ ID NO: 65, SAS, and SEQ ID NO: 66, the heavy chain complementarity-determining region comprises HCDR1, HCDR2, and HCDR3 as shown in SEQ ID NO: 67, SEQ ID NO: 68, and SEQ ID NO: 69;
or, the light chain complementarity-determining region comprises LCDR1, LCDR2, and LCDR3 as shown in SEQ ID NO: 70, RAS, and SEQ ID NO: 71, the heavy chain complementarity-determining region comprises HCDR1, HCDR2, and HCDR3 as shown in SEQ ID NO: 72, SEQ ID NO: 73, and SEQ ID NO: 74;
or, the light chain complementarity-determining region comprises LCDR1, LCDR2, and LCDR3 as shown in SEQ ID NO: 75, GTS, and SEQ ID NO: 76, the heavy chain complementarity-determining region comprises HCDR1, HCDR2, and HCDR3 as shown in SEQ ID NO: 77, SEQ ID NO: 78, and SEQ ID NO: 79.

3. The antibody according to claim 1, **characterized in that**, a light chain variable region of the antibody comprises any one of amino acid sequences shown in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, and SEQ ID NO: 21.

4. The antibody according to claim 1, **characterized in that**, a heavy chain variable region of the antibody comprises any one of amino acid sequences shown in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, and SEQ ID NO: 22.

5. The antibody according to claim 1, **characterized in that**, an amino acid sequence of a light chain variable region of the antibody is shown in SEQ ID NO: 1, an amino acid sequence of a heavy chain variable region of the antibody is shown in SEQ ID NO: 2;
or, an amino acid sequence of a light chain variable region of the antibody is shown in SEQ ID NO: 3, an amino acid sequence of a heavy chain variable region of the antibody is shown in SEQ ID NO: 4;
or, an amino acid sequence of a light chain variable region of the antibody is shown in SEQ ID NO: 5, an amino acid sequence of a heavy chain variable region of the antibody is shown in SEQ ID NO: 6;
or, an amino acid sequence of a light chain variable region of the antibody is shown in SEQ ID NO: 7, an amino acid sequence of a heavy chain variable region of the antibody is shown in SEQ ID NO: 8;
or, an amino acid sequence of a light chain variable region of the antibody is shown in SEQ ID NO: 9, an amino acid sequence of a heavy chain variable region of the antibody is shown in SEQ ID NO: 10;
or, an amino acid sequence of a light chain variable region of the antibody is shown in SEQ ID NO: 11, an amino acid sequence of a heavy chain variable region of the antibody is shown in SEQ ID NO: 12;
or, an amino acid sequence of a light chain variable region of the antibody is shown in SEQ ID NO: 13, an amino acid sequence of a heavy chain variable region of the antibody is shown in SEQ ID NO: 14;
or, an amino acid sequence of a light chain variable region of the antibody is shown in SEQ ID NO: 15, an amino acid sequence of a heavy chain variable region of the antibody is shown in SEQ ID NO: 16;
or, an amino acid sequence of a light chain variable region of the antibody is shown in SEQ ID NO: 17, an amino acid sequence of a heavy chain variable region of the antibody is shown in SEQ ID NO: 18;
or, an amino acid sequence of a light chain variable region of the antibody is shown in SEQ ID NO: 19, an amino acid sequence of a heavy chain variable region of the antibody is shown in SEQ ID NO: 20;
or, an amino acid sequence of a light chain variable region of the antibody is shown in SEQ ID NO: 21, an amino acid sequence of a heavy chain variable region of the antibody is shown in SEQ ID NO: 22.

6. The antibody according to claim 1, **characterized in that**, the light chain is formed by splicing a light chain variable region and a light chain constant region, and the heavy chain is formed by splicing a heavy chain variable region and a heavy chain constant region.

7. The antibody according to claim 6, **characterized in that**, the light chain variable region comprises a κ constant region, and the heavy chain constant region comprises IgG1, IgG2, IgG3, IgG4 or IgG1-YTE heavy chain constant region.

8. A nucleic acid, **characterized in that**, the nucleic acid encodes the antibody according to any one of claims 1-7.

9. An expression vector, **characterized in that**, the expression vector comprises the nucleic acid according to claim 8.

10. A host cell, **characterized in that**, the host cell comprises the expression vector according to claim 9.

11. A use of the antibody according to any one of claims 2 to 7 in preparation of a drug.

12. The use according to claim 11, **characterized in that**, the drug promotes BDCA-2 endocytosis, inhibits excessive activation of pDC, or inhibits pDC from generation of IFN-α by binding the anti-BDCA-2 antibody with BDCA-2.

13. A pharmaceutical composition, **characterized in that**, the pharmaceutical composition comprises at least one of the anti-BDCA-2 antibody according to any one of claims 2-7, the nucleic acid according to claim 8, the expression vector according to claim 9, or the host cell according to claim 10.

14. The pharmaceutical composition according to claim 13, **characterized in that**, the pharmaceutical composition further comprises at least one of a pharmaceutically acceptable carrier or a pharmaceutically acceptable auxiliary material.
